# EUROPEAN PATENT APPLICATION

(11) **EP 3 605 549 A1**
(43) Date of publication of application: **05.02.2020**
(21) Application number: 18777618.2
(22) Date of filing: 30.03.2018
(51) Int. Cl.: G16H 40/20, A61B 5/00

(54) **MEDICAL INFORMATION PROCESSING DEVICE AND MEDICAL INFORMATION PROCESSING METHOD**

(30) Priority: 31.03.2017 JP 2017072759
(71) Applicant: Canon Medical Systems Corporation, Otawara-shi, Tochigi 324-8550 (JP)
(72) Inventor: KANO, Yusuke, Otawara-shi Tochigi 324-8550 (JP); UTSUNOMIYA, Kazuki, Otawara-shi Tochigi 324-8550 (JP); PIAO, Longxun, Otawara-shi Tochigi 324-8550 (JP); SUGIYAMA, Shinya, Otawara-shi Tochigi 324-8550 (JP); MEGURO, Yasuyuki, Otawara-shi Tochigi 324-8550 (JP)
(74) Representative: Moreland, David
(86) International application number: PCT/JP2018/013962
(87) International publication number: WO 2018/182009

(57) **Abstract**

A medical information processing apparatus (100) according to an embodiment includes an acquiring unit (151), a classifying unit (152), and a display control unit (154). The acquiring unit (151) is configured to acquire history information representing a history of interventions provided to a subject. The classifying unit (152) is configured to classify the interventions, based on the history information. The display control unit (154) is configured to integrate and display a plurality of interventions belonging to a same class among the classified interventions.

## Description

### Field

Embodiments of the present invention relate generally to a medical information processing apparatus and a medical information processing method.

### Background

Recently, the types and the number of pieces of diagnosis data acquired in everyday practice have increased due to the advancement in medical technologies, and ways in which physicians make diagnoses or determine treatment plans by referring to the diagnosis data have also become complex. Therefore, there has been a demand for a system on which various types of diagnosis data required for a physician to make a diagnosis or to make a treatment plan are displayed in the temporal order in one screen.

### Citation List

### Patent Literature

Patent Literature 1: Japanese Patent Application Laid-open No. 2015-197737
Patent Literature 1: Japanese Patent Application Laid-open No. 2015-228103
Patent Literature 1: Japanese Patent Application Laid-open No. 2015-149103
Patent Literature 1: Japanese Patent Application Laid-open No. 2015-103220
Patent Literature 1: Japanese Patent Application Laid-open No. 2014-71592
Patent Literature 1: Japanese Patent Application Laid-open No. 2006-146820

### Summary

### Technical Problem

An object to be achieved by the present invention is to provide a medical information processing apparatus and a medical information processing method that are capable of presenting information allowing the effects of an intervention to be evaluated more appropriately. Solution to Problem

A medical information processing apparatus according to an embodiment includes an acquiring unit, a classifying unit, and a display control unit. The acquiring unit is configured to acquire history information representing a history of interventions provided to a subject. The classifying unit is configured to classify the interventions, based on the history information. The display control unit is configured to integrate and display a plurality of interventions belonging to a same class among the classified interventions.

### Brief Description of Drawings

FIG. 1 is a schematic illustrating an exemplary configuration of a medical information processing apparatus according to a first embodiment.
FIG. 2 is a schematic illustrating one example of an order table generated by an acquiring function according to the first embodiment.
FIG. 3 is a schematic illustrating one example of an intervention table generated by the acquiring function according to the first embodiment.
FIG. 4 is a schematic illustrating one example of an intervention master table generated by the acquiring function according to the first embodiment.
FIG. 5 is a schematic illustrating one example of an efficacy/effect table generated by the acquiring function according to the first embodiment.
FIG. 6 is a schematic illustrating one example of a patient table generated by the acquiring function according to the first embodiment.
FIG. 7 is a schematic illustrating one example of a response table generated by the acquiring function according to the first embodiment.
FIG. 8 is a schematic illustrating one example of an intervention classification performed by a classifying function according to the first embodiment.
FIG. 9 is a schematic illustrating one example of an intervention classification performed by the classifying function according to the first embodiment.
FIG. 10 is a flowchart illustrating a process of the intervention classification performed by the classifying function according to the first embodiment.
FIG. 11 is a flowchart illustrating a process of the intervention classification performed by the classifying function according to the first embodiment.
FIG. 12 is a schematic illustrating one example of a classification result acquired by the classifying function according to the first embodiment.
FIG. 13 is a flowchart illustrating an intervention start time and end time deriving process performed by a deriving function according to the first embodiment.
FIG. 14 is a schematic illustrating one example of the intervention start time and end time derivation performed by the deriving function according to the first embodiment.
FIG. 15 is a schematic illustrating one example of the intervention start time and end time derivation performed by the deriving function according to the first embodiment.
FIG. 16 is a schematic illustrating one example of information displayed by a display control function according to the first embodiment.
FIG. 17 is a schematic illustrating another example of the information displayed by the display control function according to the first embodiment.
FIG. 18 is a schematic illustrating another example of the information displayed by the display control function according to the first embodiment.
FIG. 19 is a schematic illustrating one example of an intervention master table according to the first modification.
FIG. 20 is a schematic illustrating one example of building of an efficacy- or effect-related classification, performed by the classifying function according to a first modification.
FIG. 21 is a schematic illustrating one example of an efficacy/effect table according to the first modification.
FIG. 22 is a schematic illustrating another example of building of an efficacy- or effect-related classification, performed by the classifying function according to the first modification.
FIG. 23 is a schematic illustrating one example of information displayed by a display control function according to a second embodiment.
FIG. 24 is a schematic illustrating another example of information displayed by the display control function according to the second embodiment.
FIG. 25 is a schematic illustrating another example of the information displayed by the display control function according to the second embodiment.
FIG. 26 is a schematic illustrating one example of a continuous duration table according to a second modification.
FIG. 27 is a flowchart illustrating an intervention start time and end time deriving process performed by a deriving function according to the second modification.
FIG. 28 is a schematic illustrating one example of information displayed by a display control function according to the second modification.

### Description of Embodiments

A medical information processing apparatus and a medical information processing method according to an embodiment will now be explained in detail with reference to some drawings.

### First Embodiment

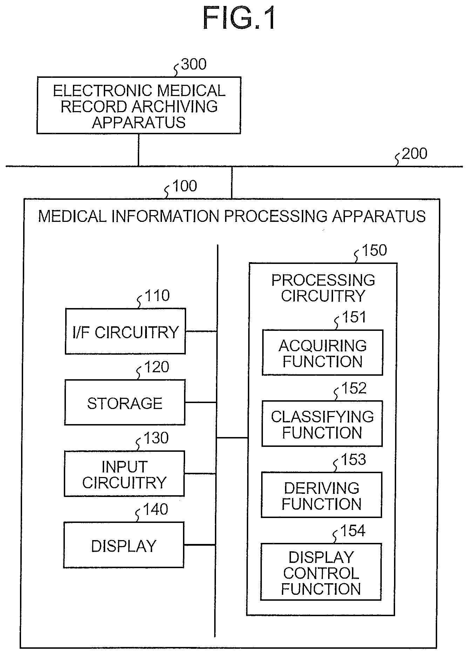

FIG. 1 is a schematic illustrating an exemplary configuration of the medical information processing apparatus according to a first embodiment.

For example, as illustrated in FIG. 1, this medical information processing apparatus 100 according to the embodiment is connected communicatively to an electronic medical record archiving apparatus 300, over a network 200. For example, the medical information processing apparatus 100 and the electronic medical record archiving apparatus 300 are installed in a hospital, for example, and are connected to each other via the network 200 such as a local area network (LAN) in the hospital.

The electronic medical record archiving apparatus 300 archives various types of diagnosis data related to the diagnoses made in the hospital, for example. For example, the electronic medical record archiving apparatus 300 is installed as a part of an electronic medical record system deployed in the hospital or the like, and archives the diagnosis data generated by the electronic medical record system. For example, the electronic medical record archiving apparatus 300 is implemented as a computer device such as a database (DB) server, and stores the diagnosis data in a storage, such as a random access memory (RAM), a semiconductor memory element such as a flash memory, a hard disk, or an optical disc.

The medical information processing apparatus 100 acquires the diagnosis data from the electronic medical record archiving apparatus 300 over the network 200, and performs various information processing using the acquired diagnosis data. For example, the medical information processing apparatus 100 is implemented as a computer device such as a workstation.

Specifically, the medical information processing apparatus 100 includes interface (I/F) circuitry 110, storage 120, input circuitry 130, a display 140, and processing circuitry 150.

The I/F circuitry 110 is connected to the processing circuitry 150, and controls transmission and communication of various types of data with the electronic medical record archiving apparatus 300. For example, the I/F circuitry 110 receives the diagnosis data from the electronic medical record archiving apparatus 300, and outputs the received diagnosis data to the processing circuitry 150. For example, the I/F circuitry 110 is implemented as a network card, a network adaptor, or a network interface controller (NIC), for example.

The storage 120 is connected to the processing circuitry 150, and stores therein various types of data. For example, the storage 120 stores therein diagnosis data received from the electronic medical record archiving apparatus 300. For example, the storage 120 is implemented as a random access memory (RAM), a semiconductor memory element such as a flash memory, a hard disk, or an optical disc, for example.

The input circuitry 130 is connected to the processing circuitry 150, and converts an input operation received from an operator into an electric signal, and outputs the result to the processing circuitry 150. For example, the input circuitry 130 is implemented as a trackball, a switch button, a mouse, a keyboard, or a touch panel.

The display 140 is connected to the processing circuitry 150, and displays various types of information and various types of image data output from the processing circuitry 150. For example, the display 140 is implemented as a liquid crystal monitor, a cathode ray tube (CRT) monitor, or a touch panel.

The processing circuitry 150 controls the elements included in the medical information processing apparatus 100, in response to input operations received from operators via the input circuitry 130. For example, the processing circuitry 150 causes the storage 120 to store therein diagnosis data outputs from the I/F circuitry 110. For example, the processing circuitry 150 reads the diagnosis data from the storage 120, and displays the diagnosis data on the display 140. For example, the processing circuitry 150 is implemented as a processor.

The overall configuration of the medical information processing apparatus 100 according to the embodiment is as explained above. With such a configuration, the medical information processing apparatus 100 according to the embodiment has a function for presenting information for allowing the effects of interventions to be evaluated more appropriately.

An "intervention" is an action performed to a subject by a health care worker such as a physician or an engineer, for the purpose of a medical treatment or a research, and is an action that can be controlled by the health care worker. Examples of an intervention include a medication, a meal, a rehabilitation, a surgical operation, an interventional radiology (IVR), and a radiotherapy treatment.

Specifically, in the embodiment, the processing circuitry 150 includes an acquiring function 151, a classifying function 152, a deriving function 153, and a display control function 154. The acquiring function 151 is one example of an acquiring unit. The classifying function 152 is one example of a classifying unit. The deriving function 153 is one example of a deriving unit. The display control function 154 is one example of a display control unit.

Each of the processing functions provided to the processing circuitry 150 is stored in the storage 120 in the format of a computer-executable program, for example. The processing circuitry 150 implements processing functions corresponding to the respective computer programs by reading the computer programs from the storage 120, and executing the read computer programs. In other words, the processing circuitry 150 having read the computer programs has the processing functions illustrated in FIG. 1.

The acquiring function 151 is configured to acquire history information representing a history of interventions provided to a subject. In the explanation hereunder, a subject will be explained as a patient.

For example, the acquiring function 151 acquires order information, intervention master information, patient information, and response information from the electronic medical record archiving apparatus 300. The acquiring function 151 then stores the acquired information in the storage 120.

The order information is information related to orders issued by an electronic medical record system or a department ordering system, for example, and includes a history of interventions provided to a patient. The intervention master information is information mapping each of the interventions, such as a medication, a meal, a rehabilitation, a surgical operation, an interventional radiology (IVR), or a radiotherapy treatment, to information related to the intervention. The patient information is information related to a patient registered in the electronic medical record system or the like. The response information is information representing a response of a patient (such as a test result or an observation) subsequent to the provision of the intervention.

For example, the acquiring function 151 generates tables by converting pieces of information acquired from the electronic medical record archiving apparatus 300 into an appropriate format, and stores the generated tables in the storage 120. In this example, the information to be included in each of such tables is acquired directly from the information archived in the electronic medical record archiving apparatus 300, but the embodiment is not limited thereto. For example, when the information included in each of such tables includes some information that cannot be acquired directly from the information archived in the electronic medical record archiving apparatus 300, the acquiring function 151 may generate the table by converting the information using a conversion table. In such a case, the conversion table is stored in the storage 120 in advance.

For example, the acquiring function 151 generates an order table and an intervention table based on the order information acquired from the electronic medical record archiving apparatus 300. Furthermore, for example, the acquiring function 151 generates an intervention master table and an efficacy/effect table based on the intervention master information acquired from the electronic medical record archiving apparatus 300. Furthermore, for example, the acquiring function 151 generates a patient table based on the patient information acquired from the electronic medical record archiving apparatus 300. Furthermore, for example, the acquiring function 151 generates a response table based on the response information acquired from the electronic medical record archiving apparatus 300.

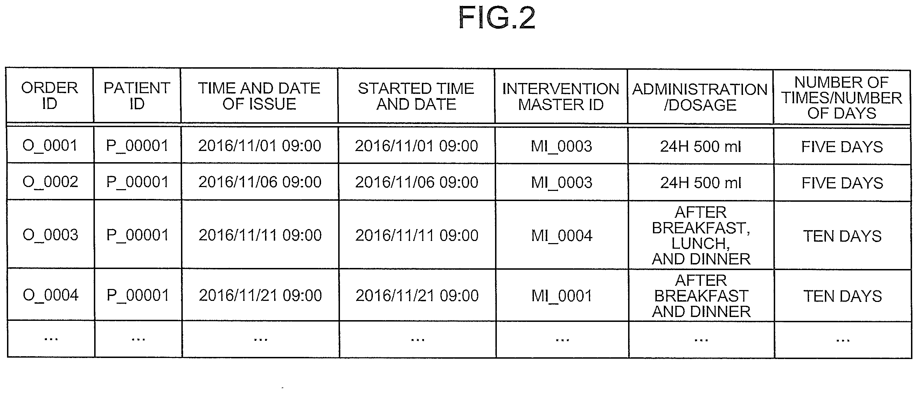

FIG. 2 is a schematic illustrating one example of the order table generated by the acquiring function 151 according to the first embodiment.

For example, as illustrated in FIG. 2, the order table includes, as data items, order ID, patient ID, time and date of issue, started time and date, intervention master ID, administration/dosage, and the number of times/the number of days. In this example, the order ID is set with identification information uniquely identifying an order. The patient ID is set with the patient information uniquely identifying a patient relevant to the order (corresponds to the patient ID set in the patient table). The time and date of issue is set with the time and the date on which the order is issued. The started time and date is set with the time and the date on which the order is started. The intervention master ID is set with identification information uniquely identifying an intervention provided to the patient as the order (corresponds to the intervention master ID set in the intervention master table). The administration/dosage is set with the administration (e.g., the timing of medication) and the dosage (e.g., the amount of medicine dosage) of the intervention provided to the patient as the order. The number of times/the number of days is set with the number of times (e.g., the number of times the medicine is administered), or the number of days (e.g., the duration of the medication) for which the intervention is provided to the patient as the order.

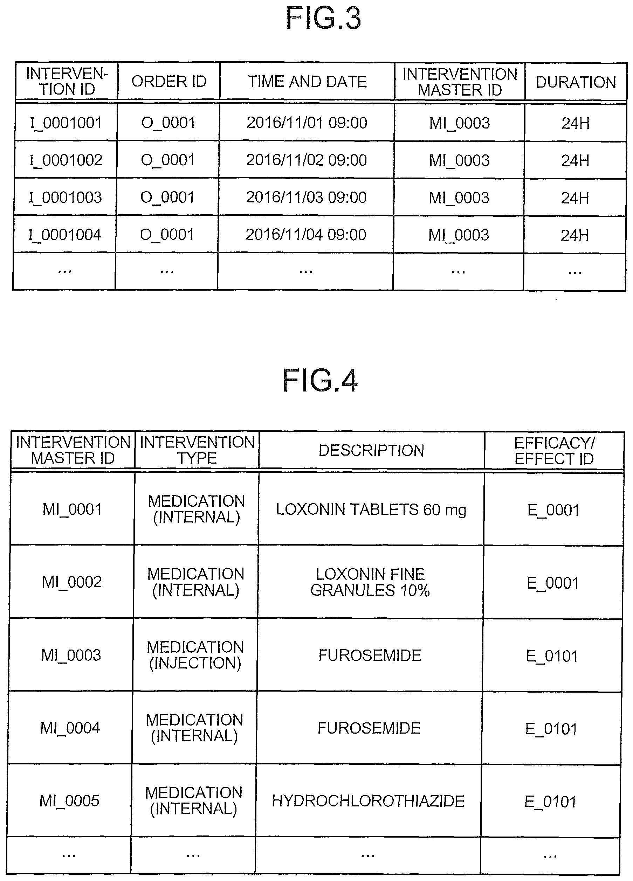

FIG. 3 is a schematic illustrating one example of the intervention table generated by the acquiring function 151 according to the first embodiment.

For example, as illustrated in FIG. 3, the intervention table includes, as data items, intervention ID, order ID, time and date, intervention master ID, and duration. The intervention ID is set with information of the interventions that are included in the order table, and are expanded in the temporal order based on the started time and date and the number of times/the number of days set in the order table. Specifically, the intervention ID is set with identification information uniquely identifying each of the interventions that are expanded in the temporal order. The order ID is set with the order ID corresponding to the intervention. The time and date is set with the time and date on which the corresponding one of the interventions having been expanded in the temporal order is started. The intervention master ID is set with the intervention master ID corresponding to the intervention. The duration is set with a duration for which corresponding one of the interventions having been expanded in the temporal order is provided.

FIG. 4 is a schematic illustrating one example of the intervention master table generated by the acquiring function 151 according to the first embodiment.

For example, as illustrated in FIG. 4, the intervention master table includes, as data items, intervention master ID, intervention type, description, and efficacy/effect ID. The intervention master ID is set with identification information uniquely identifying an intervention. The intervention type is set with information representing the type of the intervention. The description is set with information representing a description of the intervention (e.g., the name of a medicine). The efficacy/effect ID is set with identification information uniquely identifying an efficacy or an effect related to the intervention (corresponds to the efficacy/effect ID set in the efficacy/effect table).

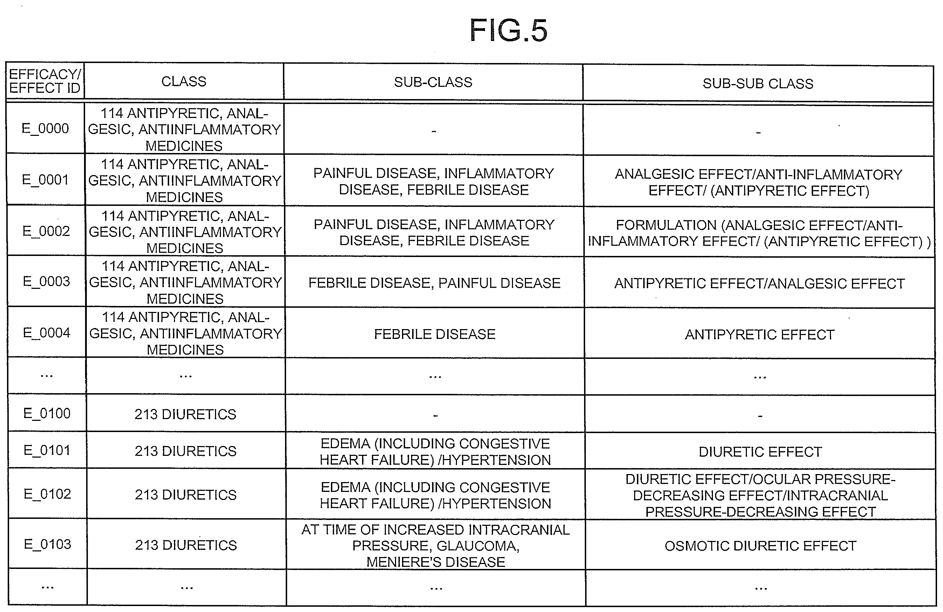

FIG. 5 is a schematic illustrating one example of the efficacy/effect table generated by the acquiring function 151 according to the first embodiment.

For example, as illustrated in FIG. 5, the efficacy/effect table includes, as data items, efficacy/effect ID, class, sub-class, and sub-sub class. The efficacy/effect table is set with information in which an efficacy or an effect related to an intervention is mapped to a plurality of classes that are different in size (class, sub-class, and sub-sub class). Specifically, the efficacy/effect ID is set with identification information that uniquely identifies an efficacy or an effect. The class is set with information representing the class of the effect corresponding to the efficacy/effect ID. The sub-class is set with information representing the sub-class of the effect corresponding to the efficacy/effect ID. The sub-sub class is set with information representing the sub-sub class of the effect corresponding to the efficacy/effect ID.

For example, when the intervention is something related to a medicine, each of the classes set to the efficacy/effect table is set based on "Drag Classification for Selection from Similar Medicines" defined by Ministry of Health, Labour and Welfare (see http://www.mhlw.go.jp/file/05-Shingikai-12404000-Hokenkyoku-Iryouka/0000029403.pdf). Furthermore, for example, each of the classes may also be set based on Generic Name Prescription Master (Ministry of Health, Labour and Welfare), Generic Medicines List (Japan Generic Medicines Association), or a medicine list or the like used in a hospital.

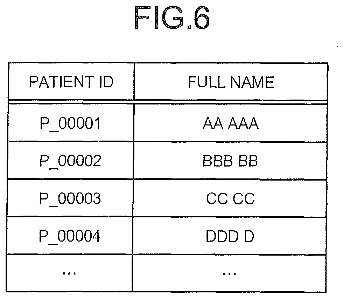

FIG. 6 is a schematic illustrating one example of the patient table generated by the acquiring function 151 according to the first embodiment.

For example, as illustrated in FIG. 6, the patient table includes, as data items, patient ID and full name. The patient ID is set with patient information uniquely identifying a patient. The full name is set with information representing the full name of the patient.

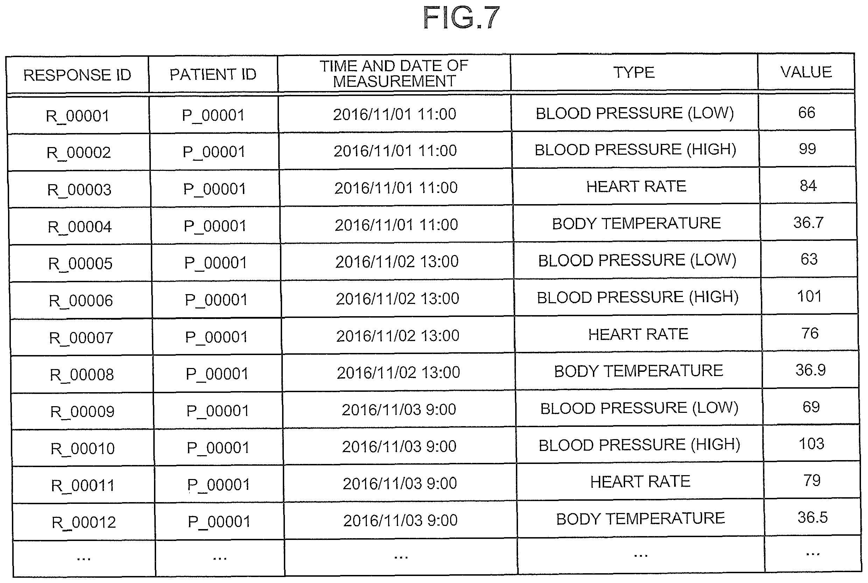

FIG. 7 is a schematic illustrating one example of the response table generated by the acquiring function 151 according to the first embodiment.

For example, as illustrated in FIG. 7, the response table includes, as data items, response ID, patient ID, time and date of measurement, type, and value. The response ID is set with identification information uniquely identifying a response. The patient ID is set with patient information uniquely identifying the patient (corresponds to the patient ID set in the patient table). The time and date of measurement is set with the time and the date on which a test value is measured as a response. The type is set with information representing the type of the test value measured as the response (e.g., blood pressure (Low: diastolic blood pressure, High: systolic blood pressure), heart rate, or body temperature). The value is set with the test value measured as the response.

For example, the process of acquiring the pieces of information from the electronic medical record archiving apparatus 300, performed by the acquiring function 151, is performed asynchronously with the processes performed by the classifying function 152, the deriving function 153, and the display control function 154, which will be explained later. The process performed by the acquiring function 151 is implemented by causing the processing circuitry 150 to read a predetermined computer program corresponding to the acquiring function 151 from the storage 120, and executing the computer program, for example.

Furthermore, explained above is an example in which the acquiring function 151 acquires the intervention master information from the electronic medical record archiving apparatus 300, but the embodiment is not limited thereto. For example, when the information stored in the intervention master table and the efficacy/effect table are not changed frequently, the system administrator or the like may manually create or update the intervention master table and the efficacy/effect table based on the intervention master information registered in the electronic medical record archiving apparatus 300, and store the intervention master table and the efficacy/effect table in the storage 120.

Referring back to FIG. 1, the classifying function 152 classifies the interventions, based on the history information acquired by the acquiring function 151. In the embodiment, the classifying function 152 classifies the interventions included in the history information acquired by the acquiring function 151 into units that are used in evaluating the effects of interventions, based on the information mapping the interventions to the information related to the interventions. The units used in evaluating the effects of interventions are, for example, orders for medicines, provisions of a medicine, medicine names, efficacies of medicines, dosages of medicine, routes of medications, instructors of the interventions, and providers of the interventions, for example.

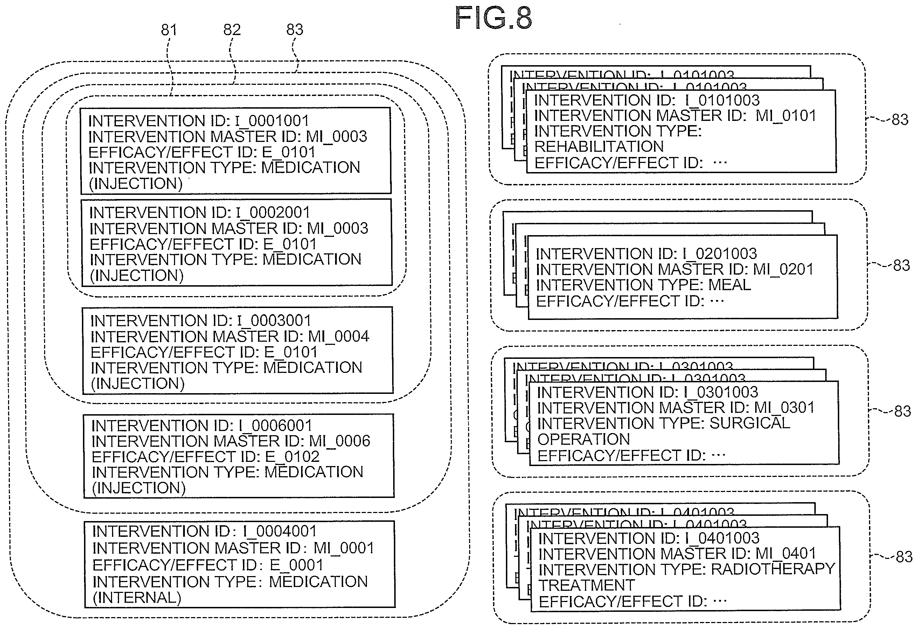

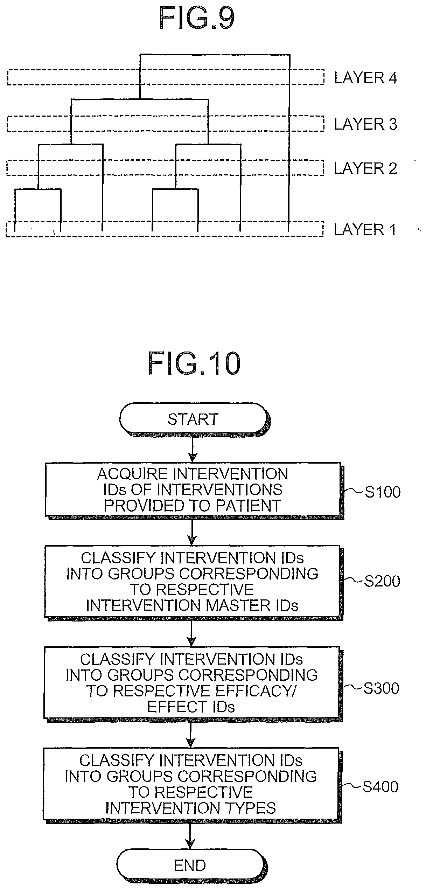

FIGS. 8 and 9 are schematics illustrating one example of the intervention classification performed by the classifying function 152 according to the first embodiment.

For example, as illustrated in FIG. 8, the classifying function 152 classifies the interventions included in the order information (the order table and the intervention table) acquired by the acquiring function 151 into a group 81 of interventions sharing the same intervention master ID, a group 82 of interventions sharing the same efficacy/effect, and a group 83 of interventions sharing the same intervention type. The classes into which the classifying function 152 groups the interventions are defined by the units used in evaluating the effects of interventions.

The classes are defined by a layer structure having layers each of which has a size that is changed in an incremental manner. For example, as illustrated in FIG. 9, the classification of interventions is defined by a plurality of layers 1 to 4, and is defined in such a manner that a higher layer represents a broader class. For example, when a class 81 that is based on the intervention master ID is defined as a "layer 1", a class 82 that is based on the efficacy/effect is defined as a "layer 2" that is at a level higher than the "layer 1", and a class 83 that is based on the intervention type is defined as a "layer 3" that is at a level higher than the "layer 2".

For example, the classifying function 152 preliminarily registers, based on information of an intervention provided to a subject, classification information to which the intervention belongs. In this case, the classifying function 152 is one example of a registering unit.

For example, the classifying function 152 defines classifications as the above-mentioned layered structure, and preliminarily registers the classifications in the storage 120, as the classification information. For example, the classifying function 152 receives units that are used in evaluating the effects of interventions from an operator through the input circuitry 130, and register the classification information based on the received units.

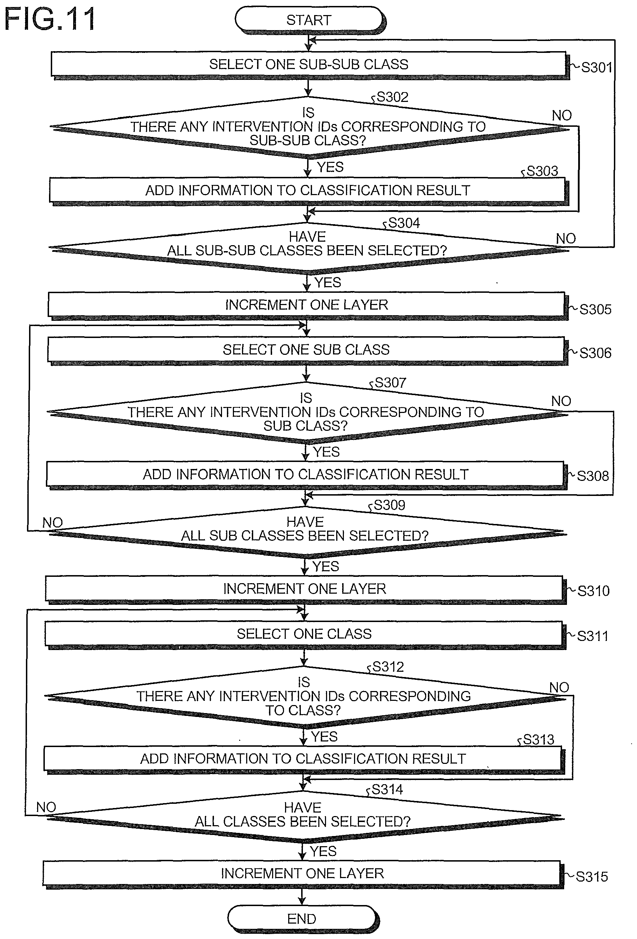

FIGS. 10 and 11 are flowcharts illustrating a process of the intervention classification performed by the classifying function 152 according to the first embodiment.

For example, as illustrated in FIG. 10, the classifying function 152 acquires intervention IDs of the interventions that are provided to a patient who is to the target of the process (Step S100).

Specifically, to begin with, the classifying function 152 refers to the order table, and identifies the order IDs that are mapped to the patient ID of the target patient. The classifying function 152 then refers to the intervention table, and acquires the intervention IDs mapped to the identified order IDs.

The classifying function 152 then classifies the acquired intervention IDs into groups of those sharing the same intervention master ID (Step S200).

Specifically, to begin with, the classifying function 152 refers to the intervention master table, and selects one of the intervention master IDs. The classifying function 152 then refers to the intervention table, and acquires the intervention IDs that are mapped to the selected intervention master ID, among those acquired at Step S100. The classifying function 152 then generates information mapping a class ID that is established in advance for each of the intervention master IDs to the group of acquired intervention IDs, as a classification result. The classifying function 152 then further refers to the intervention master table, performs the same process to each one of the intervention master IDs, and adds information mapping a class ID that is established in advance for each of the intervention master IDs to the group of intervention IDs corresponding to the intervention master ID to the classification result.

The classifying function 152 then classifies the acquired intervention IDs into groups corresponding to respective efficacy/effect IDs (Step S300).

Specifically, as illustrated in FIG. 11, to begin with, the classifying function 152 refers to the efficacy/effect table, and selects one of the sub-sub classes (Step S301). The classifying function 152 then refers to the intervention table and the intervention master table, and determines whether the intervention IDs acquired at Step S100 include any intervention ID matching the selected sub-sub class (Step S302).

If some intervention IDs match (Yes at Step S302), the classifying function 152 adds information mapping a class ID that is established in advance for each of the sub-sub classes to the group of matching intervention IDs to the classification result (Step S303). If no intervention IDs match (No at Step S302), the classifying function 152 further refers to the intervention master table, without adding any information to the classification result, and determines whether all of the sub-sub classes have been selected (Step S304).

If all of the sub-sub classes have not been selected yet (No at Step S304), the classifying function 152 selects another sub-sub class (Return to Step S301). If all of the sub-sub classes have been selected yet (Yes at Step S304), the classifying function 152 increments one layer (Step S305), and selects one of the sub-classes (Step S306) .

The classifying function 152 then refers to the intervention table and the intervention master table, and determines whether the intervention IDs acquired at Step S100 include any intervention IDs matching the selected sub-class (Step S307).

If some intervention IDs match (Yes at Step S307), the classifying function 152 adds information mapping a class ID that is established in advance for each of the sub-classes to the group of matching intervention IDs to the classification result (Step S308). If no intervention IDs match (No at Step S307), the classifying function 152 further refers to the intervention master table, without adding any information to the classification result, and determines whether all of the sub-classes have been selected (Step S309).

If all of the sub-classes have not been selected yet (No at Step S309), the classifying function 152 selects another sub-class (Return to Step S306). If all of the sub-classes have been selected (Yes at Step S309), the classifying function 152 increments one layer (Step S310), and selects one of the classes (Step S311).

The classifying function 152 then refers to the intervention table and the intervention master table, and determines whether the intervention IDs acquired at Step S100 include any intervention IDs matching the selected class (Step S312).

If some intervention IDs match (Yes at Step S312), the classifying function 152 adds information mapping a class ID that is established in advance for each of the classes to the group of matching intervention IDs to the classification result (Step S313). If no intervention ID match (No at Step S312), the classifying function 152 further refers to the intervention master table, without adding any information to the classification result, and determines whether all of the classes have been selected (Step S314).

If all of the classes have not been selected yet (No at Step S314), the classifying function 152 selects another class (Return to Step S310). If all of the classes have already been selected (Yes at Step S314), the classifying function 152 increments one layer (Step S315), and ends the process of classifying the intervention IDs in units of efficacy/effect IDs.

Referring back to FIG. 10, the classifying function 152 classifies the acquired intervention ID into groups corresponding to the respective intervention types (Step S400).

Specifically, to begin with, the classifying function 152 refers to the intervention master table, and selects one of the intervention types. The classifying function 152 then refers to the intervention table, and acquires the intervention IDs corresponding to the selected intervention type from the intervention IDs acquired at Step S100. The classifying function 152 then generates information mapping a class ID that is established in advance for each of the intervention types to the group of acquired intervention IDs, as a classification result. The classifying function 152 further refers to the intervention master table, performs the same process for all of the intervention types, and adds information mapping a class ID that is established in advance for each of the intervention types to the group of intervention IDs corresponding to the intervention master ID to the classification result.

The process from Steps S100 to S400 and Steps S301 to S315 are implemented by causing the processing circuitry 150 to read a predetermined computer program corresponding to the classifying function 152 from the storage 120, and to execute the computer program, for example.

Explained in the process illustrated in FIG. 10 is an example in which the processes are performed in the order of Step S200, Step S300, and Step S400, but the order in which these processes are performed is not limited thereto, and the order may be swapped.

Furthermore, explained above is an example in which the classifying function 152 classifies the interventions in units of an intervention master ID, units of a class, units of a sub-class, and units of a sub-sub class of the efficacy or effects, and units of an intervention type, but the embodiment is not limited thereto, and the classifying function 152 may classify the interventions to any classification units other than those classes.

For example, the classifying function 152 may classify the interventions in units of a medicine. There are some medicines containing different amounts of the same active ingredient, such as Loxonin tablets 60g and Loxonin tablets 30g. To evaluate the effects of interventions related to such medicines containing different amount of one active ingredient, there are cases in which it is preferable to evaluate the effect of these medicines as one unit. In such a case, by classifying the interventions in units of a medicine, it is possible to evaluate the effects of the interventions more appropriately.

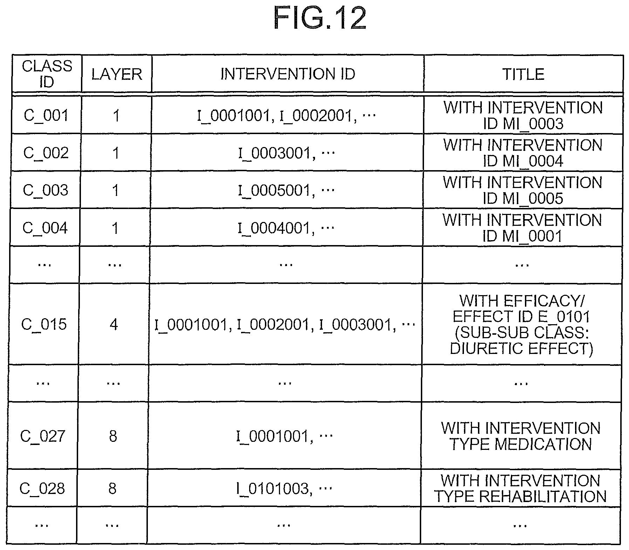

FIG. 12 is a schematic illustrating one example of a classification result acquired by the classifying function 152 according to the first embodiment.

For example, as illustrated in FIG. 12, the classification result acquired by the classifying function 152 includes, as data items, class ID, layer, intervention ID, and title. The class ID is set with a class ID that is established in advance for each of intervention master IDs included in the intervention master table, each of the classes, the sub-classes, and sub-sub classes included in the efficacy/effect table, and each of the intervention types included in the intervention master table. The layer is set with a value indicating the layer defined for a group represented by the class ID. The intervention ID is set with a set of intervention IDs that are mapped with the class ID. The title is set with information representing a description for the class ID.

Explained herein is an example in which a plurality of interventions are grouped into a class, but the embodiment is not limited thereto. For example, the classifying function 152 may classify one intervention into one unit, or classify one intervention into a plurality of units.

For example, when one intervention master ID is mapped to only one intervention (intervention ID) in the order information (the order table and the intervention table), the classifying function 152 classifies the intervention as one unit. In the same manner, for example, when there is some efficacy/effect ID or intervention type that is mapped to only one intervention (intervention ID), the classifying function 152 classifies the intervention as one unit.

Furthermore, for example, when one intervention (intervention ID) is mapped with a plurality of intervention master IDs in the order information (the order table and the intervention table), the classifying function 152 classifies the intervention into a plurality of units. In the same manner, for example, if one intervention (intervention ID) is mapped with a plurality of efficacy/effect IDs or a plurality of intervention types, the classifying function 152 classifies the intervention into a plurality of units.

For example, the classifying function 152 integrates a plurality of interventions belonging to the same classification information and manage the integrated interventions as one unit. In this case, the classifying function 152 is one example of a managing unit.

In the embodiment, the classifying function 152 integrates the interventions which have been performed repeatedly with time and manage the integrated interventions as the one unit. For example, the classifying function 152 stores classification result obtained by the above-mentioned process as a table in the storage 120. The table of the classification result is referred and used by the deriving function 153 and the display control function 154 which are described below.

Referring back to FIG. 1, the deriving function 153 groups a series of interventions included in the history information acquired by the acquiring function 151, based on the temporal continuity of the interventions.

For example, based on a classification result from the classifying function 152, the deriving function 153 derives the start time and the end time of a series of interventions included in the history information acquired by the acquiring function 151. Explained in the embodiment is an example in which the deriving function 153 derives the started time and date as the start time, and the ended time and date as the end time.

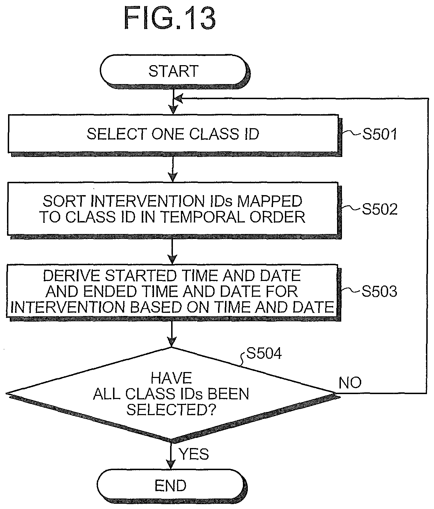

FIG. 13 is a flowchart illustrating an intervention start time and end time deriving process performed by the deriving function 153 according to the first embodiment.

For example, as illustrated in FIG. 13, the deriving function 153 refers to the classification result acquired by the classifying function 152 (see FIG. 12), and selects one of the class IDs (Step S501).

The deriving function 153 then refers to the classification result acquired by the classifying function 152, acquires intervention IDs mapped to the selected class ID, and sorts the acquired intervention IDs in the temporal order (Step S502).

The deriving function 153 then derives the started time and date and the ended time and date for each of the intervention IDs having been sorted in the temporal order, based on the time and date, the intervention master ID, and the duration set in the intervention table (Step S503) .

At this time, the deriving function 153 refers to the time and date, the intervention master ID, and the duration that are mapped to each of the intervention IDs having been sorted in the temporal order, sequentially in the temporal order, and derives the started time and date and the ended time and date, for the intervention, based on predetermined conditions.

At this time, for example, the deriving function 153 determines that a series of intervention has ended when the intervention master ID changes. Furthermore, for example, the deriving function 153 determines that a series of intervention has ended when a cycle of morning-noon-evening is broken, in the time and date. Furthermore, for example, the deriving function 153 determines that a series of intervention has ended when the duration changes.

Every time the deriving function 153 determines that the intervention has ended, the deriving function 153 derives the started time and date and the ended time and date, for the series of interventions up to that point in time, as one unit.

When these conditions are used, for example, the deriving function 153 may be configured not to determine that intervention has ended if the duration is continuing although the intervention master ID has changed. Furthermore, for example, the deriving function 153 may be configured not to determine that intervention has ended if a cycle of morning-noon-evening in the time and date is continuing although the intervention master ID has changed. Furthermore, for example, the deriving function 153 may be configured not to determine that intervention has ended if the cycle of morning-noon-evening in the time and date is broken only once (e.g., with only one morning lacking).

The deriving function 153 then further refers to the intervention table, and determines whether all of the class IDs have been selected (Step S504).

If all of the class IDs have not been selected yet (No at Step S504), the deriving function 153 selects another class ID (Return to Step S501). If all of the class ID have been selected (Yes at Step S504), the deriving function 153 ends the process of deriving the started time and date and the ended time and date of interventions.

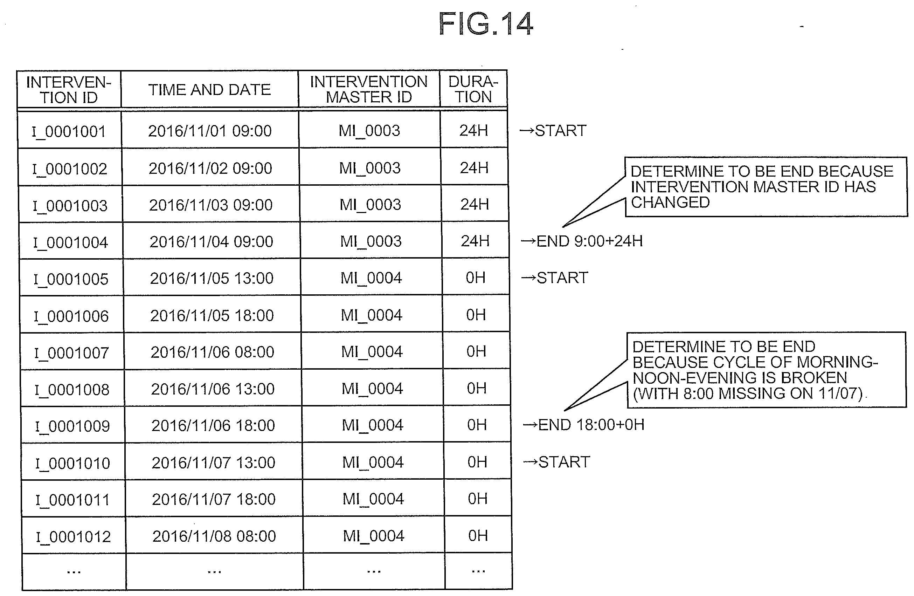

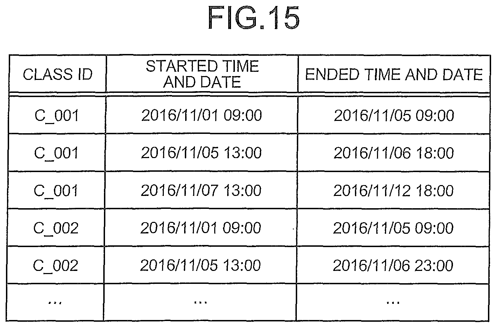

FIGS. 14 and 15 are schematics illustrating one example of intervention start time and end time derivation performed by the deriving function 153 according to the first embodiment.

For example, as illustrated in FIG. 14, in this example, among intervention IDs "I_0001001" to "I_0001012" that are arranged in the temporal order, an intervention master ID changes from "MI_0003" to "MI_0004" between "I_0001004" and "I_0001005". In such a case, the time and date "2016/11/01 09:00" corresponding to the first intervention ID "I_0001001", among the intervention IDs "I_0001001" to "I_0001004" with the intervention master ID "MI_0003", serves as the started time and date of these interventions. The time and date "2016/11/05 09:00", resultant of adding a duration of 24H to the time and date "2016/11/04 09:00" corresponding to the last intervention ID "I_0001004", among the intervention IDs "I_0001001" to "I_0001004" with the intervention master ID "MI_0003", serves as the ended time and date of these interventions.

As another example, among the intervention IDs "I_0001001" to "I_0001012" that are arranged in the temporal order, the cycle of morning-noon-evening is broken once between "I_0001009" and "I_0001010" (with the morning (8:00) missing on 11/07). In such a case, among the intervention IDs "I_0001005" to "I_0001009" in which the cycles of morning-noon-evening are continuous, the time and date "2016/11/05 13:00" corresponding to the first intervention ID "I_0001005" serves as the started time and date of these interventions. The time and date "2016/11/06 18:00" resultant of adding a duration of 0H to the time and date "2016/11/06 18:00" corresponding to the last intervention ID "I_0001009", among the intervention IDs "I_0001005" to "I_0001009" in which the cycles of morning-noon-evening are continuous, serves as the ended time and date of these interventions.

As a result, for example, as illustrated in FIG. 15, for the class ID "C_001", "2016/11/01 09:00" is derived as the started time and date, and "2016/11/05 09:00" is derived as the ended time and date of a series of interventions. Also for the class ID "C_001", "2016/11/05 13:00" is derived as the started time and date, and "2016/11/06 18:00" is derived as the ended time and date of the next series of interventions.

Referring back to FIG. 1, the display control function 154 integrates and displays a plurality of interventions belonging to a same class among the classified interventions. In the embodiment, the display control function 154 integrates and display the interventions which have been performed repeatedly with time. Here, the interventions which have been performed repeatedly with time include interventions which have been repeated continuously and interventions which have been repeated intermittently.

The display control function 154 displays information representing the interventions included in the history information acquired by the acquiring function 151 in the units classified by the classifying function 152.

In the embodiment, the display control function 154 displays the information in the units grouped by the deriving function 153.

For example, the display control function 154 displays information representing the start time and the end time of a series of interventions, derived by the deriving function 153. The display control function 154 also displays information representing a response of a patient subsequent to the provision of an intervention included in the history information acquired by the acquiring function 151, in a manner mapped to the information representing the intervention.

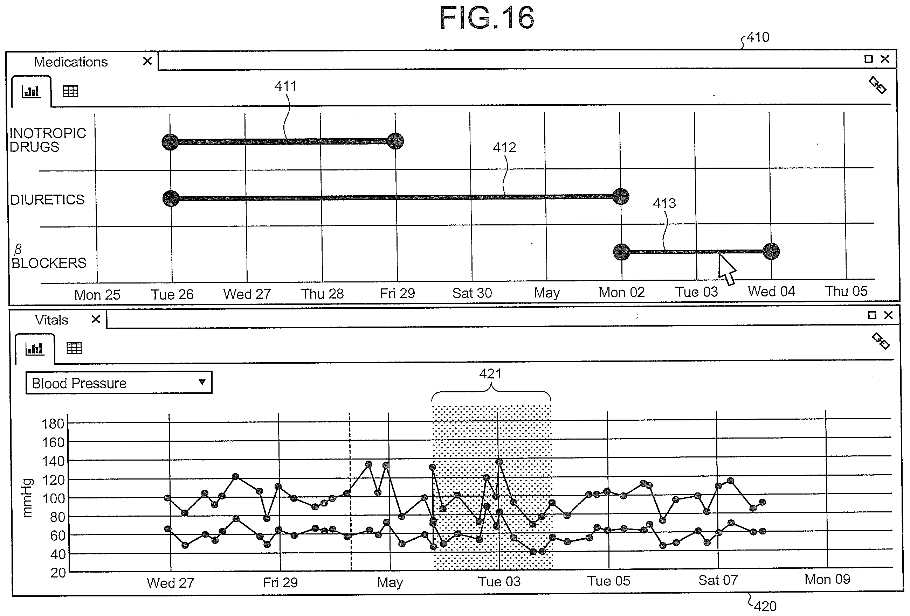

FIG. 16 is a schematic illustrating one example of the information displayed by the display control function 154 according to the first embodiment.

For example, as illustrated in FIG. 16, the display control function 154 displays an intervention display area 410 for displaying information related to interventions, and a response display area 420 for displaying information related to responses on the display 140, on top of each other. The intervention display area 410 and the response display area 420 both have an X axis (axis in the horizontal direction) and a Y axis (an axis in the vertical direction), and these horizontal axes in these areas represent time and date.

The display control function 154 then displays the information representing the started time and date and the ended time and date included in the units classified, in the intervention display area 410, based on the classification result acquired by the classifying function 152. For example, as illustrated in FIG. 16, the display control function 154 displays, for each unit of interventions related to medicines having the same efficacy/effect, a line connecting the started time and date and the ended time and date, in the same row. In this example, among the three lines 411 to 413 illustrated in FIG. 16, the first line 411 from the top indicates the started time and date and the ended time and date of interventions "inotropic drugs", and the second line 412 from the top indicates the started time and date and the ended time and date for the interventions "diuretics". The third line 413 from the top represents the started time and date and the ended time and date for the interventions "β-blockers".

The display control function 154 also refers to the response table and the patient table, and acquires information related to the responses of the target patient (such as the time and date on which a test value is measured, the type of the test value, and the test value), and displays the acquired information on the response display area 420. For example, as illustrated in FIG. 16, the display control function 154 refers to the time and date of measurement, the type, and the value that are set in the response table, and displays the blood pressure values of the target patient in the temporal order.

In this manner, by causing the display control function 154 to display the information representing the start time and the end time of interventions in the units used in evaluating the effects of interventions, it is possible to make a series of interventions visually easy to understand. In this manner, operators can easily evaluate the effect of interventions.

At this time, the display control function 154 displays the information representing the responses corresponding to a time range matching the duration of the intervention.

For example, the display control function 154 receives an operation of selecting one of the lines connecting the started time and date and the ended time and date, displayed in the intervention display area 410 from the operator, via the input circuitry 130. Upon receiving the operation, the display control function 154 displays the range from the started time and date and to the ended time and date corresponding to the line selected by the operator, in a manner distinguishable from the other ranges in the response display area 420. For example, as illustrated in FIG. 16, when the line 413 related to the interventions "β-blockers" is selected, the display control function 154 displays the range 421 from the started time and date to the ended time and date corresponding to the selected line 413 in an emphasized manner, using a color different from that of the other ranges, in the response display area 420.

In this manner, by causing the display control function 154 to display a response of the patient subsequent to the provision of an intervention, in a manner mapped to the information representing the intervention, operators can easily compare the intervention with the resultant response, for each unit of the interventions. Furthermore, the operators can easily recognize whether the intervention actually has had an effect, so that the operator can determine the treatment or therapy to be provided next, appropriately.

Furthermore, for example, when the classifying function 152 classifies one intervention into a plurality of units, the display control function 154 may display the units in a visually distinguishable manner.

In such a case, for example, the display control function 154 may change how the line connecting the started time and date and the ended time and date is displayed, or display a mark in the middle of the line connecting the started time and date and the ended time and date, so that a plurality of units are simultaneously represented.

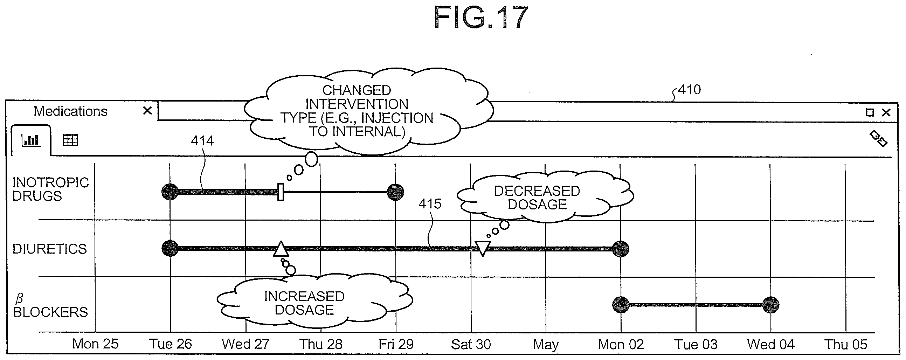

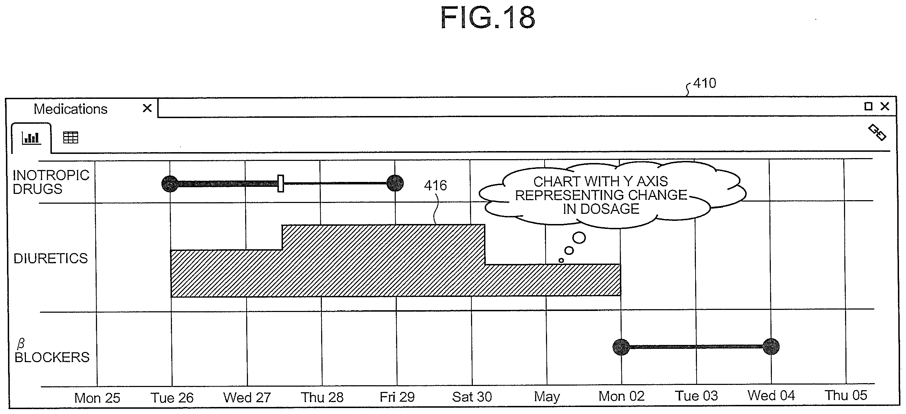

FIGS. 17 and 18 are schematics illustrating other examples of the information displayed by the display control function 154 according to the first embodiment.

For example, in order to visualize the timing at which the intervention type is changed from "injections" to "oral administrations", as indicated by a line 414 related to the interventions "inotropic drugs" in FIG. 17, the display control function 154 changes the display modes of the line connecting the started time and date and the ended time and date (e.g., thickness or color of the line) from the timing thereon. Furthermore, for example, in order to visualize the timing at which the medicine dosage is changed, the display control function 154 displays a mark indicating the timing at which the medicine dosage is changed (e.g., the triangle and the inverted triangle) on the line connecting the started time and date and the ended time and date, as indicated on a line 415 related to the interventions "diuretics" illustrated in FIG. 17. At this time, for example, the display control function 154 displays different marks for the timing at which the medicine dosage is increased, and the timing at which the medicine dosage is reduced (e.g., marks with different colors or shapes).

Furthermore, for example, the display control function 154 may also display the line connecting the started time and date and the ended time and date as a chart having a Y axis representing a change in the medicine dosage, as indicated by the line 416 related to the interventions "diuretics" illustrated in FIG. 18. In such a case, the display control function 154 changes the width of the line in the Y-axis direction at the timing at which the medicine dosage is changed, and changes the size of the width of the line in the Y-axis direction in such a manner that the medicine dosage at each timing is represented thereby.

Furthermore, for example, the display control function 154 may also change the way in which the line is displayed in an emphasized manner using the size of or the color of a mark, depending on how much medicine dosage is changed, or on the degree of change in the efficacy or the effect (e.g., a change to a powerful medicine).

As described above, in the first embodiment, the classifying function 152 classifies the interventions into units that are used in evaluating the effects of interventions, based on the information mapping the interventions to the information related to the intervention. The display control function 154 then displays the information representing the interventions in the classified units, based on the classification result from the classifying function 152. Therefore, according to the first embodiment, it is possible to present information for allowing the effects of interventions to be evaluated more appropriately.

For example, among conventional technologies, there has been a method for assisting comparison between a medication and the resultant test value by displaying marks or the like at the timing at which the medicine is administered, or on a duration for which the medicine is administered, side by side with a graph of test result values. With such a conventional technology, upon displaying the duration for which the medicine is administered, interventions are generally grouped in units that are classified from the viewpoint of data processing, or of improving the operation efficiency, e.g., in units of an order or a medicine type. However, such units of comparison do not necessary meet the criteria for those to be used in evaluating the effects of interventions. For example, when evaluated is the effects of administrations of inotropic drugs, as a treatment for cardiac failures, and if such interventions are not in the same order, or if different types of inotropic drug are used, these interventions may be handled as having been done over different durations, and prevented from being compared against the resultant effects over a duration that is appropriate for the evaluation of the effects of the interventions.

By contrast to such a conventional technology, according to the embodiment described above, because information representing interventions is displayed in the units used in evaluating the effects of interventions, the operator can evaluate the effects of an intervention more appropriately.

### First Modification

Presented in the embodiment described above is an example in which one intervention (intervention master ID) is set with one efficacy/effect ID in the intervention master table, as illustrated in FIG. 4, but the embodiment is not limited thereto. For example, one intervention (intervention master ID) may be set with a plurality of efficacy/effect IDs in the intervention master table.

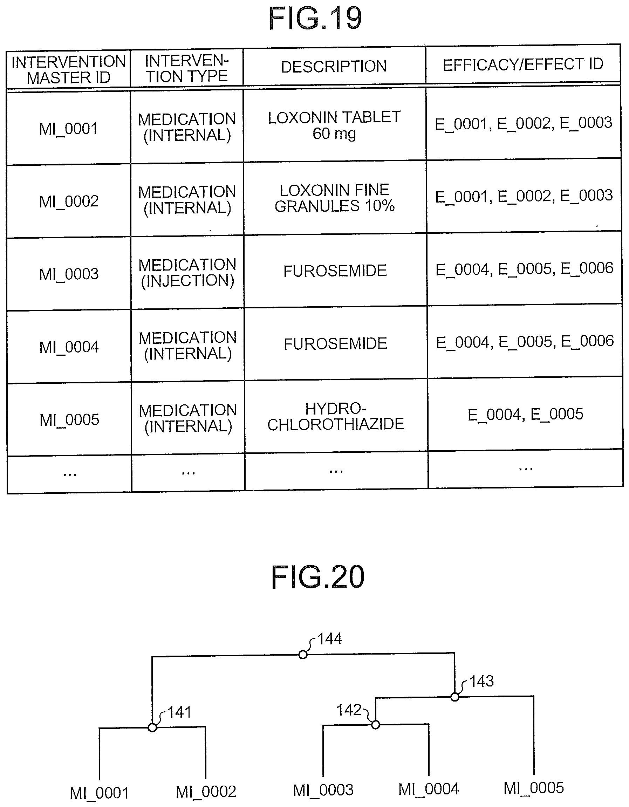

FIG. 19 is a schematic illustrating one example of an intervention master table according to a first modification.

For example, as illustrated in FIG. 19, one intervention (intervention master ID) is set with a plurality of efficacy/effect IDs related to the intervention in the intervention master table.

In such a case, the classifying function 152 builds a plurality of classes related to the efficacies or the effects using clustering, based on the efficacy/effect IDs set in the intervention master table. The classifying function 152 then classifies the interventions included in the order information (the order table and the intervention table) based on the build classes.

FIG. 20 is a schematic illustrating one example of building of a classification related to the efficacies or effects, performed by the classifying function 152 according to the first modification. At this time, illustrated in FIG. 20 is an example in which the intervention master table illustrated in FIG. 19 is used.

For example, as illustrated in FIG. 20, the classifying function 152 builds one sub-sub class 141 from a group of interventions having the efficacy/effect ID including "E_0001", "E_0002", and "E_0003" (the intervention master IDs "MI_0001", "MI_0002"). The classifying function 152 also builds one sub-sub class 142 from a group of interventions having the efficacy/effect ID including "E_0004", "E_0005", and "E_0006" (intervention master IDs "MI_0003", "MI_0004"). Furthermore, the classifying function 152 builds one sub-class 143 from a group of interventions having the efficacy/effect ID including "E_0004" and "E_0005" (intervention master IDs "MI_0003" to "MI_0005"). The classifying function 152 also builds one class 144 from a group of interventions having the identification information set to the efficacy/effect ID including "E_00" (the intervention master IDs "MI_0001" to "MI_0005").

Furthermore, explained in the embodiment described above is an example in which the efficacy/effect table includes a plurality of classes (class, sub-class, sub-sub class), as data items, as illustrated in FIG. 5, but the embodiment is not limited thereto. For example, the efficacy/effect table may include an item containing a description of the efficacy/effect as a data item, instead of the classes built in advance.

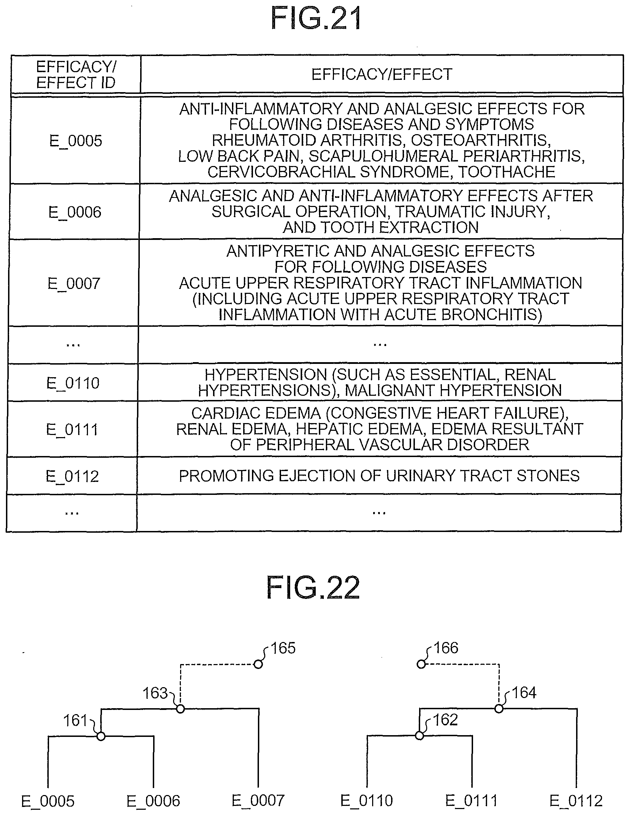

FIG. 21 is a schematic illustrating one example of the efficacy/effect table according to the first modification.

For example, as illustrated in FIG. 21, the efficacy/effect table includes an item that is set with information representing the description of an efficacy/effect (efficacy/effect) as a data item, instead of the class, the sub-class, and the sub-sub class built in advance.

In such a case, the classifying function 152 builds a plurality of classes, such as class, sub-class, and sub-sub class using clustering, based on the words or sentences included in the description of the efficacy/effect set in the intervention master table. The classifying function 152 then classifies interventions included in the order information (the order table and the intervention table) based on the build classes.

FIG. 22 is a schematic illustrating another example of building of an efficacy- or effect-related classification, performed by the classifying function 152 according to the first modification. The example illustrated in FIG. 22 is an example in which the efficacy/effect table illustrated in FIG. 21 is used.

For example, as illustrated in FIG. 22, the classifying function 152 builds one sub-sub class 161 based on the description of the efficacy/effect having the efficacy/effect ID set to "E_0005", and on the description of the efficacy/effect having the efficacy/effect ID set to "E_0006". The classifying function 152 also builds one sub-sub class 162 based on the description of the efficacy/effect having the efficacy/effect ID set to "E_0110", and on the description of the efficacy/effect having the efficacy/effect ID set to "E_0111". The classifying function 152 also builds one sub-class 163 based on the description of the efficacy/effect having the efficacy/effect ID set to "E_0005", the description of the efficacy/effect having the efficacy/effect ID set to "E_0006", and the description of the efficacy/effect having the efficacy/effect ID set to "E_0007". The classifying function 152 also builds one sub-class 164 based on the description of the efficacy/effect having the efficacy/effect ID set to "E_0110", the description of the efficacy/effect having the efficacy/effect ID set to "E_0111", and the description of the efficacy/effect having the efficacy/effect ID set to "E_0112". The classifying function 152 also builds one class 165 based on the description of the efficacy/effect having the efficacy/effect ID set to "E_0005", the description of the efficacy/effect having the efficacy/effect ID set to "E_0006", the description of the efficacy/effect having the efficacy/effect ID set to "E_0007", and descriptions of the efficacy/effects having other efficacy/effect IDs. The classifying function 152 also builds one class 166 based on the description of the efficacy/effect having the efficacy/effect ID set to "E_0110", the description of the efficacy/effect having the efficacy/effect ID set to "E_0111", the description of the efficacy/effect having the efficacy/effect ID set to "E_0112", and the descriptions of the efficacy/effects having other efficacy/effect IDs.

In the manner described above, according to the first modification, because the classifying function 152 builds a plurality of classes using clustering, it is no longer necessary to retain data by defining a plurality of classes in advance. Therefore, the workload in the table building can be reduced.

### Second Embodiment

Explained in the embodiment above is an example in which the information representing interventions is displayed in units that are classified based on the information mapping the interventions to the information related to the interventions, but the embodiment is not limited thereto.

For example, the display control function 154 may be configured to switch the units for displaying the information representing the interventions, based on some conditions that are designated by an operator. Such an example will now be explained, as a second embodiment. In the second embodiment, the difference with the embodiment described above will be mainly explained, and redundant explanations with the embodiment described above will be omitted.

For example, the display control function 154 switches the granularity related to the size of the classes of interventions.

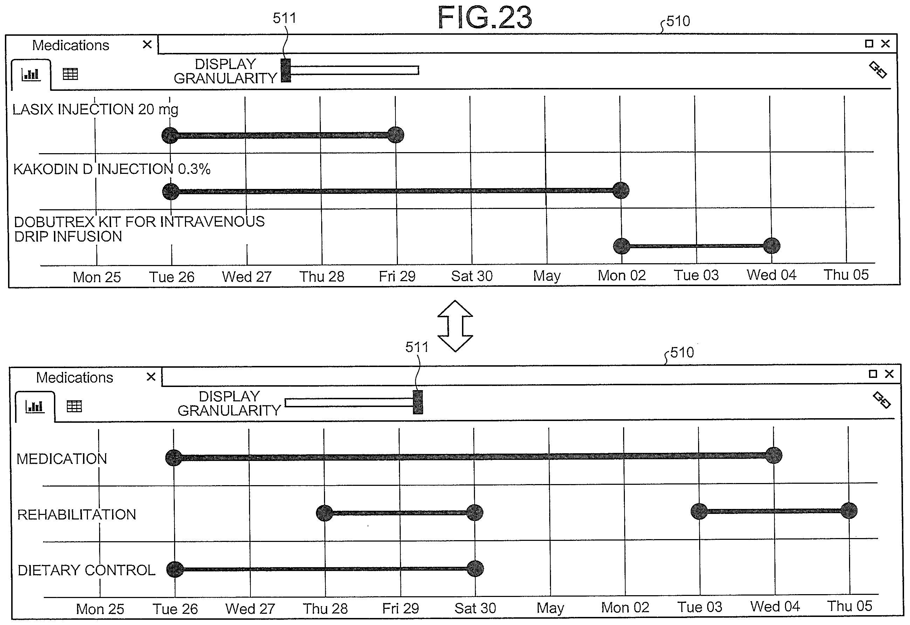

FIG. 23 is a schematic illustrating one example of the information displayed by the display control function 154 according to the second embodiment.

For example, as illustrated in FIG. 23, the display control function 154 displays a slide bar 511 for receiving an operation for designating the granularity related to the size of the intervention classes to be displayed in the intervention display area 510. When an operator makes an operation of moving the slide bar 511, the display control function 154 displays the information representing the interventions in the unit of classes in the granularity corresponding to the position of the slide bar 511. Examples of the units of classes include the product name, the generic name, the efficacy or the effect of a medicine, the intervention type, and the presence or absence of any interventions. The granularity related to the size of classes becomes coarser in the order listed herein.

For example, as the slide bar 511 is moved further toward the left, the display control function 154 uses more granular classes as the units for displaying the interventions, and as the slide bar 511 is moved further toward the right, the display control function 154 uses less granular classes as the units for displaying the interventions. For example, in the top example illustrated in FIG. 23, as a result of the slide bar 511 being moved to the left, the information representing interventions is displayed in units of interventions that are classified based on the class of product names. Furthermore, in the bottom example illustrated in FIG. 23, as a result of the slide bar 511 being moved to the right, the information representing interventions is displayed in the units of interventions classified based on the class of intervention types.

Illustrated in FIG. 23 is an example in which the slide bar 511 is used, but the graphical user interface (GUI) for receiving an operation for changing the granularity is not limited thereto, and radio buttons or checkboxes may also be used, as some examples.

For example, the display control function 154 uses "large", "medium", and "small" as the sizes of the class of the efficacies or effects, displays radio buttons corresponding to the respective classes, and receives an operation on one of the radio buttons from an operator. When an operation is made on the radio button "large", the display control function 154 displays the information representing the interventions in units of a class. When an operation is made on the radio button "medium", the display control function 154 displays the information representing the interventions in units of a sub-class. When an operation is made on the radio button "small", the display control function 154 displays the information representing the interventions in units of a sub-sub class.

Furthermore, as an example, the display control function 154 displays, for a plurality of intervention types, checkboxes corresponding to the respective intervention types, such as "medication", "rehabilitations", "dietary management", ..., and receives an operation corresponding to one of the checkboxes from an operator. The display control function 154 then displays the information representing the interventions in units of the intervention type for which the checkbox is operated by the operator.

In the manner described above, by causing the display control function 154 to switch the granularity related to the size of the classes of interventions, the units in which the interventions are displayed can be switched based on the granularity related to the size of the classes.

Furthermore, for example, the display control function 154 may be enabled to switch the granularity related to the duration of interventions.

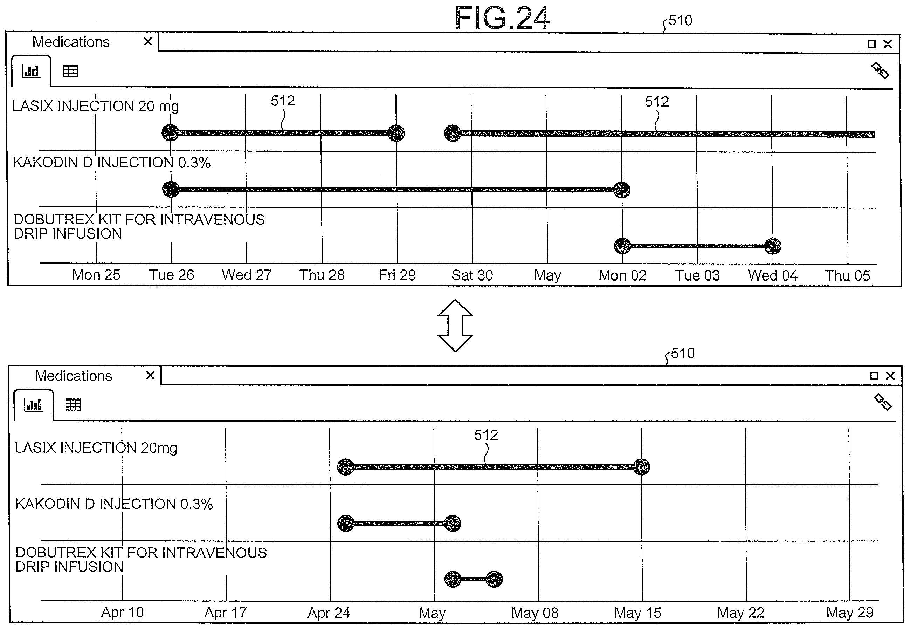

FIG. 24 is a schematic illustrating another example of the information displayed by the display control function 154 according to the second embodiment.

For example, as illustrated in FIG. 24, the display control function 154 receives an operation for designating the range of time and date to be displayed in the intervention display area 510. When such an operation is made by an operator, the display control function 154 sets the unit of the time and date in which the interventions are to be displayed in the intervention display area 510 in such a manner that the designated range of time and date is displayed. Examples of the unit of time and date include the units of a day, a week, and a month. For example, in the top example in FIG. 24, the time and date is displayed in units of a day. In the bottom example, the time and date is displayed in units of a week.

In accordance with the unit of time and date displayed in the intervention display area 510, the display control function 154 changes the granularity of duration in which the interventions are displayed in the intervention display area 510. For example, as indicated by the lines 512 related to the intervention "Lasix Injection 20 mg" illustrated in FIG. 24, while the display control function 154 displays lines indicating the started time and date and the ended time and date correspondingly to different interventions in units of a day, the display control function 154 displays only one line, in an integrated manner, in the display in units of a week, by ignoring the break with a duration less than one day.

In this manner, by causing the display control function 154 to switch the granularity related to the duration of interventions, it is possible to change the units in which the interventions are displayed based on the granularity in the temporal order. In this manner, without causing the operator to explicitly adjust the granularity in units of classes, the display of the interventions can be switched to that having an appropriate granularity.

Furthermore, for example, when the classifying function 152 classifies an intervention into a plurality of units, the display control function 154 may be configured to display the intervention classified into a plurality of units that are designated by an operator as one, among the units.

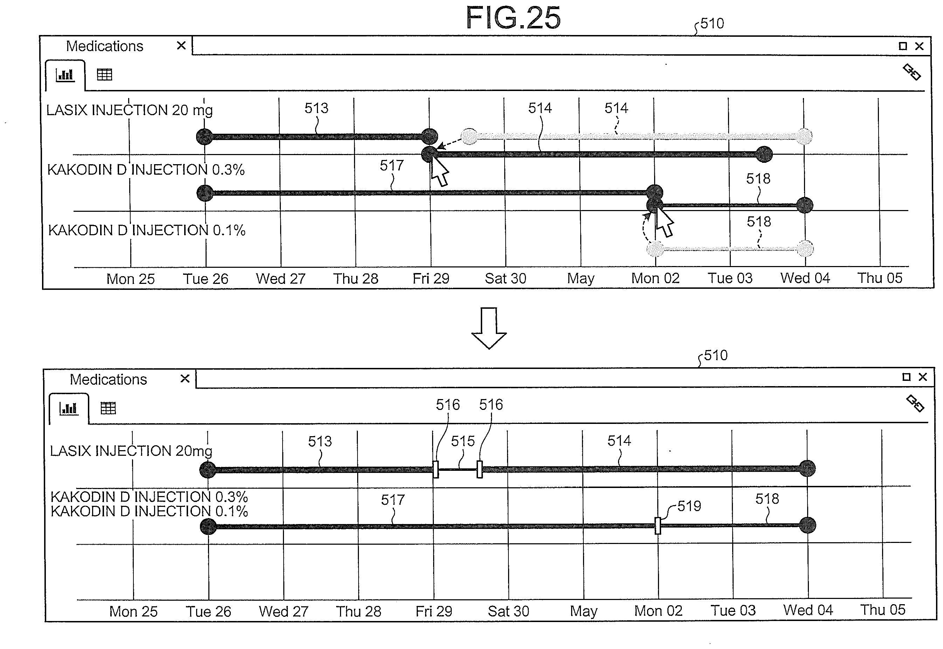

FIG. 25 is a schematic illustrating another example of the information displayed by the display control function 154 according to the second embodiment.

For example, as illustrated in FIG. 25, the display control function 154 receives an operation for connecting the ended time and date of one intervention to the started time and date of another intervention, both interventions being displayed in the intervention display area 510. When an operator makes such an operation, the display control function 154 connects the durations of the two interventions, for which the operation is received, and display the result on the same row.

For example, for the two durations related to the same intervention (the started time and date to the ended time and date), examples of which are durations of the intervention "Lasix Injection 20 mg" illustrated in the top diagram in FIG. 25, the display control function 154 receives an operation (such as a dragging operation of a mouse) for bringing the started time and date included in a line 514 that represents the other duration, to the same position as that of the ended time and date included in a line 513 that represents the one duration. In such a case, for example, if there is any break in the connection between the line 513 representing one duration and the line 514 that represents the other duration, the display control function 154 displays these two durations related to the same intervention in a connected manner, by displaying a new line 515 corresponding to the broken period, as illustrated for the intervention of "Lasix Injection 20 mg" in the bottom diagram in FIG. 25. Furthermore, for example, the display control function 154 displays marks 516 at the position of the ended time and date included in the line 513 that represents the one duration, and the position of the started time and date included in the line 514 that represents the other duration, both of these time and dates being included in the respective lines before these durations are connected.

Furthermore, for example, for the durations related to two interventions, an example of which is the durations of the interventions of "Kakodin D Injection 0.3%" and "Kakodin D Injection 0.1%" illustrated in the top diagram in FIG. 25, the display control function 154 receives an operation (such as a dragging operation of a mouse) for bringing the started time and date included in the line 518 that represents the other duration, to the position of the ended time and date included in the line 517 that represents the one duration. In such a case, for example, the display control function 154 displays the durations related to the two respective interventions in a connected manner, by connecting the ended time and date included in the line 517 that represents the one duration with the started time and date included in the line 518 that represents the other duration, as illustrated for the intervention "Kakodin D Injection 0.3%" in the bottom diagram in FIG. 25. Furthermore, for example, the display control function 154 displays a mark 519 at a position where the ended time and date included in the line 517 that represents the one duration is connected to the started time and date included in the line 518 that represents the other duration.

At this time, for example, the display control function 154 may also be configured to receive an operation designating to disconnect the connected durations from the operator, and to display the durations as originally having been displayed, by disconnecting the durations having been connected once, in response to the operation.

In this manner, by causing the display control function 154 to display a plurality of units designated by an operator as one unit, the operator can edit the units for displaying the information related to interventions, in the manner desirable to the operator.

As described above, in the second embodiment, the display control function 154 switches the units for displaying the information representing interventions based on the conditions designated by an operator. Therefore, according to the second embodiment, the operator can dynamically change the units for displaying the information representing interventions.

### Second Modification

Furthermore, explained in the embodiment described above is an example in which the two durations related to the same intervention are displayed in a manner connected to each other, in response to an operation received from an operator, as illustrated in FIG. 25, but the embodiment is not limited thereto. For example, a table in which a continuous duration for allowing broken durations to be handled as one duration may be stored in the storage 120, and two durations may be displayed in a connected manner based on the continuous duration set in the table.

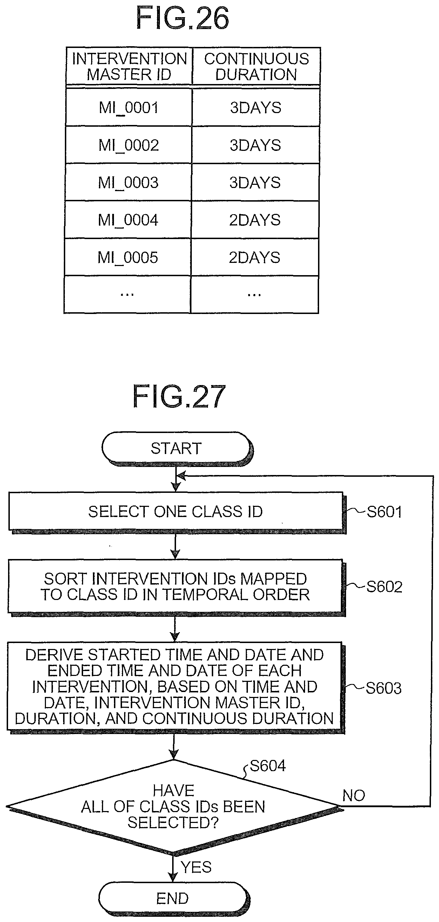

FIG. 26 is a schematic illustrating one example of such a continuous duration table according to the second modification.

For example, as illustrated in FIG. 26, the continuous duration table includes, as data items, intervention master ID and continuous duration. The intervention master ID is set with identification information uniquely identifying an intervention (corresponds to the intervention master ID set in the intervention master table). The continuous duration is set with the length of an intermittent period allowing durations to be considered as one duration, when there is such an intermittent period between the durations related to the intervention.

Explained herein is an example of the continuous duration table in which a continuous duration is set for each intervention master ID, but the embodiment is not limited thereto. For example, a continuous duration may be set to each efficacy/effect ID, each patient ID, each order ID or each intervention ID, or a continuous duration may be set for a combination of any of these IDs in the continuous duration table.

In such a case, the deriving function 153 further refers to the continuous duration set in the continuous duration table, and derives the started time and date and the ended time and date, for a series of interventions included in the history information acquired by the acquiring function 151.

FIG. 27 is a flowchart illustrating an intervention start time and end time deriving process performed by the deriving function 153 according to the second modification.

For example, as illustrated in FIG. 27, the deriving function 153 refers to the classification result acquired by the classifying function 152 (see FIG. 12), and selects one of the class IDs (Step S601).

The deriving function 153 then refers to the classification result acquired by the classifying function 152, acquires the intervention IDs mapped to the selected class ID, and sorts the acquired intervention IDs in the temporal order (Step S602).

The deriving function 153 then derives, for each of the intervention IDs arranged in the temporal order, the started time and date and the ended time and date for each of the interventions, based on the time and date, the intervention master ID, and the duration that are set in the intervention table, and the continuous duration set in the continuous duration table (Step S603).

At this time, the deriving function 153 refers to the time and date, the intervention master ID, and the duration that are mapped to each of the intervention IDs arranged in the temporal order, in the temporal order, and derives the started time and date and the ended time and date for the corresponding intervention based on predetermined conditions.

At this time, for example, the deriving function 153 determines that a series of intervention has ended if the intervention master ID has changed, and the intermittent period in the intervention is longer than the continuous duration set in the continuous duration table. Furthermore, for example, the deriving function 153 determines that a series of intervention has ended if the cycle of morning-noon-evening in the time and date is broken, and the intermittent period in the intervention is longer than the continuous duration set in the continuous duration table. Furthermore, for example, the deriving function 153 determines that a series of intervention has ended if the duration has changed, and the intermittent period in the intervention is longer than the continuous duration set in the continuous duration table.

Every time the deriving function 153 determines that the intervention has ended, the deriving function 153 derives the started time and date and the ended time and date for the intervention, in units of a series of interventions.

The deriving function 153 then further refers to the intervention table, and determines whether all of the class IDs have been selected (Step S604).

If all of the class IDs have not been selected yet (No at Step S604), the deriving function 153 selects another class ID (Return to Step S601). If all of the class IDs have been selected (Yes at Step S604), the deriving function 153 ends the process of deriving the started time and date and the ended time and date for the interventions.

The display control function 154 then displays information representing the started time and date and the ended time and date, for a series of interventions, derived by the deriving function 153, as the information representing interventions.

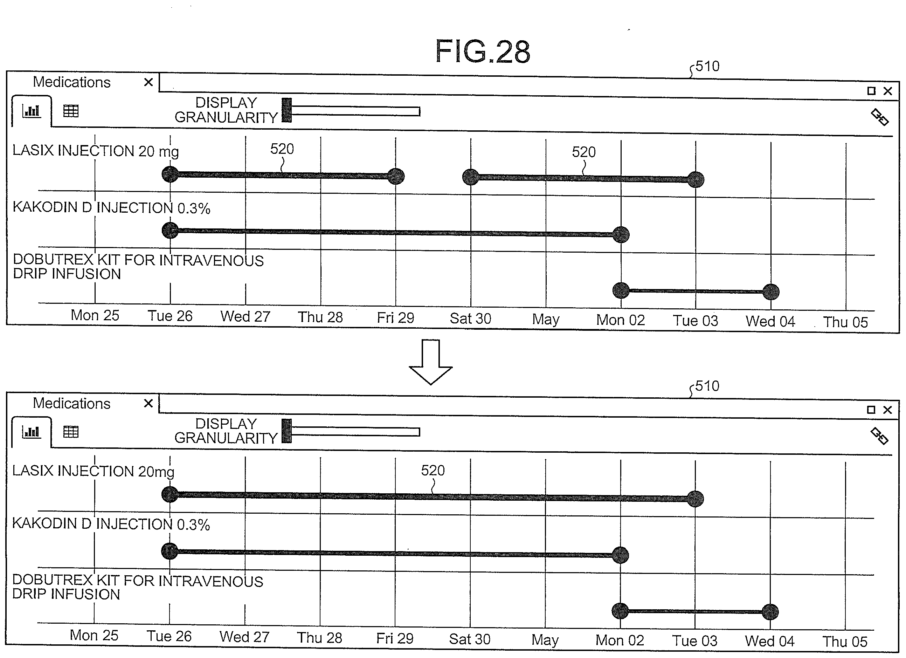

FIG. 28 is a schematic illustrating one example of information displayed by the display control function 154 according to the second modification. The top example in FIG. 28 gives an example in which the started time and date and the ended time and date of each of the interventions are derived without using the continuous duration explained above, and the bottom example gives an example in which the started time and date and the ended time and date of each of the interventions are derived using the continuous duration explained above.

For example, as illustrated in the top diagram in FIG. 28, when the started time and date and the ended time and date of each of the interventions are derived without using the continuous duration, a line 520 representing the duration related to one intervention may be displayed broken. To address this issue, in this modification, if the intermittent period within the duration related to one intervention is equal to or shorter than the continuous duration set in the continuous duration table, the line 520 representing the duration related to the intervention is displayed as one line without any break, as in the bottom diagram in FIG. 28, for example.

Depending on the intervention type, there may be some cases in which it is preferable to handle durations with an intermittent period as one duration, in evaluating the effect of the intervention. For example, for the interventions related to medicines, a period for which the effect or efficacy of one medicine is continuing is sometimes handled as a duration of the medicine in evaluating the effect. In such a case, in the second modification, when there is any intervention related to medicines, a period in which the effect or the efficacy of the medicine is continuing can be handled as one duration by setting a continuous duration by which the effect or the efficacy of a medicine is lost, in the continuous duration table.

Furthermore, explained in the embodiment described above is an example in which the processing functions described above are implemented as one piece of processing circuitry 150, but the embodiment is not limited thereto. For example, the processing circuitry 150 may be configured as a combination of a plurality of independent processors, and the processing functions are implemented by causing each of the processors to execute a corresponding computer program. The processing functions provided to the processing circuitry 150 may be implemented in a manner distributed to or integrated into one or more pieces of processing circuitry, as appropriate.

The term "processor" used in the explanation above means circuitry such as a central processing unit (CPU), a graphics processing unit (GPU), an application specific integrated circuit (ASIC), a programmable logic device (such as a simple programmable logic device (SPLD) or a complex programmable logic device (CPLD)), and a field programmable gate array (FPGA)). The processor implements a function by reading a computer program stored in the storage 120, and executing the computer program. Instead of storing the computer program in the storage 120, the computer program may be configured to be embedded directly in the processor circuitry. In such a case, the processor implements a function by reading the computer program embedded in the circuitry, and executing the computer program. The processor in the embodiment is not limited to the configuration in which each processor is configured as one piece of circuitry, but one processor may be configured as a combination of a plurality of independent pieces of circuitry, and caused to implement the functions.

The computer program executed by the processor is provided in a manner incorporated in a read-only memory (ROM) or storage, for example, in advance. The computer program may also be provided in a manner recorded in a computer-readable recording medium, such as a compact disc read-only memory (CD-ROM), a flexible disk (FD), a compact disc recordable (CD-R), and a digital versatile disc (DVD), as a file in a format installable to or executable on these devices. The computer program may also be provided or distributed by storing the computer program in a computer connected to a network such as the Internet, and making available for downloading over the network. The computer program has a modular structure including the functional units described above, for example. As actual hardware, by causing a CPU to read the computer program from a recording medium such as a ROM, and to execute the computer program, such modules are loaded onto the main memory device, and are generated on the main memory device.

According to at least one of the embodiments described above, it is possible to present information allowing the effects of an intervention to be evaluated more appropriately.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the inventions.

## Claims

1. A medical information processing apparatus comprising:
an acquiring unit configured to acquire history information representing a history of interventions provided to a subject;
a classifying unit configured to classify the interventions, based on the history information; and
a display control unit configured to integrate and display a plurality of interventions belonging to a same class among the classified interventions.

2. The medical information processing apparatus according to claim 1, wherein the display control unit is configured to integrate and display the interventions which have been performed repeatedly with time.

3. The medical information processing apparatus according to claim 1 or 2, further comprising: a deriving unit configured to derive start time and end time of a series of interventions included in the history information based on a classification result, wherein
the display control unit is configured to display control unit is configured to display information representing the start time and the end time of the series of interventions.

4. The medical information processing apparatus according to claim 1 or 2, wherein the display control unit is further configured to switch units for displaying the information representing the interventions, based on a condition designated by an operator.

5. The medical information processing apparatus according to claim 4, wherein the display control unit is further configured to switch granularity related to size of a class of the interventions.

6. The medical information processing apparatus according to claim 4, wherein the display control unit is further configured to switch granularity related to a duration of the interventions.

7. The medical information processing apparatus according to claim 4, wherein the display control unit is further configured to, when the interventions are classified into a plurality of units, group units, among the units, that are designated by an operator into one unit, and display the one unit.

8. The medical information processing apparatus according to claim 1 or 2, wherein the display control unit is further configured to, when the interventions are classified into a plurality of units, display the units in a visually distinguishable manner.

9. The medical information processing apparatus according to claim 1 or 2, wherein the display control unit is further configured to display information representing a response corresponding to an intervention included in the history information, in a manner mapped to the information representing the intervention.

10. The medical information processing apparatus according to claim 9, wherein the display control unit is further configured to display information representing a response corresponding to a time range matching a duration of the corresponding intervention.

11. The medical information processing apparatus according to claim 1 or 2, wherein the interventions are at least one of a medication, a meal, a rehabilitation, a surgical operation, an interventional radiology (IVR), and a radiotherapy treatment.

12. The medical information processing apparatus according to claim 1 or 2, wherein units into which the interventions are classified are at least one of an order for a medicine, provisioning of a medicine, a name of a medicine, an efficacy of a medicine, a dosage of a medicine, a method of medication, an instructor of the corresponding intervention, and a provider of the corresponding intervention.

13. A medical information processing apparatus comprising:
a registering unit configured to register, based on information of an intervention provided to a subject, classification information to which the intervention belongs; and
a managing unit configured to integrate a plurality of interventions belonging to a same classification information and manage the integrated interventions as one unit.

14. The medical information processing apparatus according to claim 13, wherein the managing unit is configured to integrate the interventions which have been performed repeatedly with time and manage the integrated interventions as the one unit.

15. A medical information processing method comprising:
acquiring history information representing a history of interventions provided to a subject;
classifying the interventions, based on the history information; and
integrating and displaying a plurality of interventions belonging to a same class among the classified interventions.

16. A medical information processing method comprising:
registering, based on information of an intervention provided to a subject, classification information to which the intervention belongs; and
integrating a plurality of interventions belonging to a same classification information and managing the integrated interventions as one unit.
